# EUROPÄISCHE PATENTANMELDUNG

(11) **EP 0 990 991 A1**
(43) Veröffentlichungstag der Anmeldung: **05.04.2000**
(21) Anmeldenummer: 98118424.5
(22) Anmeldetag: 29.09.1998
(51) Int. Cl.: G06F 15/02, G06F 17/60, G06F 19/00

(54) **Vorrichtung zur Ernährungskontrolle**

(71) Anmelder: Menzel, Ute, 80687 München (DE); Schlinkmann, Reinhard, 80687 München (DE)
(72) Erfinder: Menzel, Ute, 80687 München (DE); Schlinkmann, Reinhard, 80687 München (DE)
(74) Vertreter: Banzer, Hans-Jörg, Dipl.-Ing.

(57) **Zusammenfassung**

Vorrichtung zur automatischen Ernährungskontrolle, wobei über eine Tastatur (3) persönliche Daten eines Benutzers, wie z.B. sein Alter, Geschlecht oder Gewicht usw., sowie Lebensmitteldaten, die von dem Benutzer verzehrte Lebensmittel beschreiben, eingebbar sind. Eine Steuereinheit (2) summiert den eingegebenen Lebensmitteldaten entsprechende ernährungsspezifische Kennwerte, wie z.B. die entsprechende Kalorienzahl und/oder Fettmenge, über ein bestimmtes Zeitintervall, insbesondere über einen Tag, auf und bestimmt somit eine Gesamtkalorien- und/oder Gesamtfettmenge, die mit einem von den persönlichen Daten des Benutzers abgeleiteten Grenzwert verglichen wird. Bei Erreichen des Grenzwertes wird eine Warnmeldung an den Benutzer ausgegeben. Die erfindungsgemäße Vorrichtung läßt sich insbesondere zur Diätkontrolle eines Benutzers einsetzen, um den täglichen Kalorien- und/oder Fettverzehr des Benutzers zu überwachen.

## Beschreibung

Die vorliegende Erfindung betrifft eine Vorrichtung zu Ernährungskontrolle, insbesondere eine Vorrichtung zur persönlichen Ernährungskontrolle, mit deren Hilfe die durch den Verzehr von Lebensmitteln über einen bestimmten Zeitraum, beispielsweise während eines Tages, verzehrte Gesamtmenge bestimmter ernährungsspezifischer Kennwerte, wie z.B. die gesamte Kalorien- und/oder Fettmenge, kontrolliert werden kann.

Eine zuverlässige Ernährungskontrolle ist aus verschiedenen Gründen von Bedeutung. Zum einen kann sie Personen, die ihr Gewicht halten oder ein bestimmtes Wunschgewicht erreichen wollen, durch Überwachung der Kalorienzahl und/oder der Fettmenge der verzehrten Lebensmittel bei der Überwachung des Gewichts oder einer Diät unterstützen. Des weiteren ist eine derartige Ernährungskontrolle auch aus medizinischen Gründen sinnvoll, um den Nährstoffgehalt oder dergleichen der verzehrten Lebensmittel zu überwachen. Eine zuverlässige Ernährungskontrolle ist jedoch für die jeweilige Person nur äußerst aufwendig möglich, da sie von der Person selbst durchgeführt werden muß und lediglich anhand von Ernährungstabellen möglich ist, die zu verschiedenen Lebensmitteln den Nährstoffgehalt (z.B. Fett-, Eiweiß-, Kohlenhydrate- oder Vitamingehalt) oder Energiegehalt (kcal oder kJ) ausweisen. Die Person muß somit für jedes verzehrte Lebensmittel selbst Buch führen und aus den Tabellen die den verzehrten Lebensmittel entsprechende Fettmenge oder Kalorienzahl usw. ablesen und beispielsweise tageweise aufsummieren. Eine stets aktualisierte Erfassung der Fettmenge bzw. Kalorienzahl wird jedoch insbesondere dann erschwert, wenn sich die entsprechende Person an verschiedenen Orten aufhält. Darüber hinaus muß die Person einen vorgegebenen Grenzwert für die im Laufe eines Tages angesammelte Kalorienzahl oder Fettmenge selbst überwachen, um beispielsweise ein vorgegebenes Wunschgewicht halten oder erreichen zu können.

Der vorliegenden Erfindung liegt daher die Aufgabe zugrunde, eine Vorrichtung zur Ernährungskontrolle bereit zu stellen, die einen Benutzer bei der Ernährungskontrolle unterstützt und insbesondere automatisch bestimmte ernährungsspezifische Kennwerte, wie z.B. die Kalorienzahl und/oder die Fettmenge, welche beispielsweise während eines Tages aufgrund des Verzehrs von Lebensmittel angesammelt werden, zu überwachen.

Diese Aufgabe wird gemäß der vorliegenden Erfindung durch eine Vorrichtung mit den Merkmalen des Anspruches 1 gelöst. Die Unteransprüche beschreiben bevorzugte und vorteilhafte Ausführungsformen der vorliegenden Erfindung, die u.a. die Benutzerfreundlichkeit unterstützen und zu einem möglichst einfachen Aufbau der erfindungsgemäßen Vorrichtung beitragen.

Die erfindungsgemäße Vorrichtung sieht vor, daß von einem Benutzer einerseits persönliche Daten, wie z.B. das Alter, das Geschlecht, das Gewicht und/oder den Lebensstil betreffende Angaben, und andererseits Lebensmitteldaten, welche insbesondere die Art und die Anzahl der von dem Benutzer verzehrten Lebensmittel beschreiben können, eingegeben werden. Die Vorrichtung enthält eine Steuerung, die anhand der eingegebenen persönlichen Daten sowie der eingegebenen Lebensmitteldaten über ein bestimmtes Zeitinvervall die Summe der jeweils interessierenden ernährungsspezifischen Kennwerte, wie insbesondere die in den verzehrten Lebensmitteln enthaltene Kalorienzahl und/oder Fettmenge, bildet, um somit eine ernährungsspezifische Kennwertsumme (wie z.B. eine Gesamtkalorienzahl und/oder Gesamtfettmenge) zu berechnen. Des weiteren vergleicht die Steuerung die somit berechnete ernährungsspezifische Kennwertsumme mit einem bestimmten Grenzwert, der insbesondere aus den persönlichen Daten abgeleitet worden ist und beispielsweise derart gewählt wird, daß er über einen bestimmten Beobachtungszeitraum zu einem von dem Benutzer angestrebten Wunschgewicht führt. Wird der zuvor erläuterte Grenzwert von der berechneten ernährungsspezifischen Kennwertsumme erreicht, wird eine Warnung an den Benutzer ausgegeben, was beispielsweise mittels eines akustischen Warntons erfolgen kann.

Wie bereits erläutert worden ist, kann mit Hilfe der vorliegenden Erfindung insbesondere die Kalorienzahl und Fettmenge der verzehrten Lebensmittel überwacht werden. Die vorliegende Erfindung ist jedoch nicht auf dieses bevorzugte Anwendungsgebiet beschränkt, sondern kann prinzipiell überall dort eingesetzt werden, wo bestimmte ernährungsspezifische Kennwerte der verzehrten Lebensmittel aus verschiedenen Gründen überwacht werden sollen. Somit kann beispielsweise die vorliegende Erfindung auch zur Überwachung oder Kontrolle der während eines Tages zu sich genommenen Vitaminmenge bestimmter Vitamine oder der den verzehrten Lebensmitteln entsprechende Cholesteringehalt aus medizinischen Gründen automatisch überwacht werden.

Die Eingabe der Lebensmitteldaten ist beispielsweise anhand einer gespeicherten Vorschlagsliste von Lebensmitteln möglich, die durch eigene Angaben, d.h. zusätzlich und bisher nicht gespeicherte Lebensmittel, erweitert werden kann. Diese Vorschlagsliste ist insbesondere Teil einer Lebensmitteldatenbank, in der pro Einheit eines bestimmten Lebensmittels die dazugehörigen Kalorien- und Fettangaben usw. abgelegt sind. Nach der Eingabe eines in einem bestimmten Zeitraum zu erreichenden Gewichts berechnet die erfindungsgemäße Vorrichtung vorzugsweise die täglich maximal zu verzehrende Kalorienzahl und/oder Fettmenge. Die tägliche Eingabe von sportlichen Aktivitäten, welche zu einer Reduzierung der aufsummierten Kalorienzahl führen, ist möglich, wobei dies wiederum vorzugsweise in Verbindung mit einer Sportdatenbank erfolgt, in der zu bestimmten sportlichen Aktivitäten der jeweils entsprechende Kalorienverbrauch gespeichert ist. Auch diese Sportdatenbank ist durch den Benutzer erweiterbar.

Die erfindungsgemäße Vorrichtung ermöglicht nicht nur die Ernährungskontrolle, sondern dient auch zu bestimmten Informationszwecken, da die in den zuvor beschriebenen Lebensmittel- und Sportdatenbanken gespeicherten Einträge von dem Benutzer abgerufen und somit eine 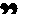 was-wäre-wenn"-Überprüfung durchgeführt werden kann, um den Verzehr bestimmter Lebensmittel oder bestimmte sportliche Aktivitäten im voraus planen zu können. Darüber hinaus kann die vorliegende Erfindung gemäß einem bevorzugten Ausführungsbeispiel Tages-, Wochen-, oder Jahresbilanzen über die Kalorienzahl und/oder Fettmenge der verzehrten Lebensmittel zur Verfügung stellen, die von dem Benutzer abfragbar sind.

Die vorliegende Erfindung ist vorzugsweise in Form eines Meinen und tragbaren Gerätes ausgestaltet, welches in jede Handtasche paßt, so daß ein mobiles Gerät geschaffen wird, welches beispielsweise von Hausfrauen zur Unterstützung ihrer Diät stets bei sich getragen werden kann. Das Gerät ist leicht bedienbar und wird insbesondere in Verbindung mit einem Handbuch angeboten, wobei das Gerät derart aufklappbar ist, daß im Geräteunterteil der eigentliche Rechner und im Geräteoberteil bzw. Gerätedeckel das Handbuch angeordnet ist und der Benutzer während der Bedienung des Gerätes jederzeit über das integrierte Handbuch Bedienerinformationen ablesen kann. Das Gerät kann somit auch von EDV-Laien ohne weiteres bedient werden.

Insgesamt ermöglicht die vorliegende Erfindung eine Kontrolle und eine Verbesserung des persönlichen Eßverhaltens, ohne daß umfangreiche Lebensmitteltabellen oder sogar eine ärztliche Unterstützung erforderlich ist.

Die vorliegende Erfindung wird nachfolgend unter Bezugnahme auf die beigefügte Zeichnung anhand eines bevorzugten Ausführungsbeispiels näher beschrieben.
Fig. 1 zeigt ein vereinfachtes Blockschaltbild des bevorzugten Ausführungsbeispiels der vorliegenden Erfindung,
Fig. 2 zeigt eine perspektivische Ansicht des in Fig. 1 gezeigten Ausführungsbeispiels, und
Fig. 3 zeigt ein Flußdiagramm zur Erläuterung des Betriebs des in Fig. 1 und Fig. 2 dargestellten Ausführungsbeispiels.

Die vorliegende Erfindung wird nachfolgend anhand eines bevorzugten Ausführungsbeispiels der vorliegenden Erfindung beschrieben, welches zur Überwachung der Kalorienzahl und/oder Fettmenge der von einem Benutzer verzehrten Lebensmittel eingesetzt wird. Wie jedoch bereits erwähnt worden ist, ist die vorliegende Erfindung nicht auf dieses Anwendungsgebiet beschränkt, sondern kann überall dort eingesetzt werden, wo beliebige ernährungsspezifische Kennwerte der verzehrten Lebensmittel, wie z.B. auch der Cholesteringehalt oder der Anteil bestimmter Vitamine während eines bestimmten Zeitraumes, wie z.B. eines Tages, insbesondere mobil überwacht werden soll.

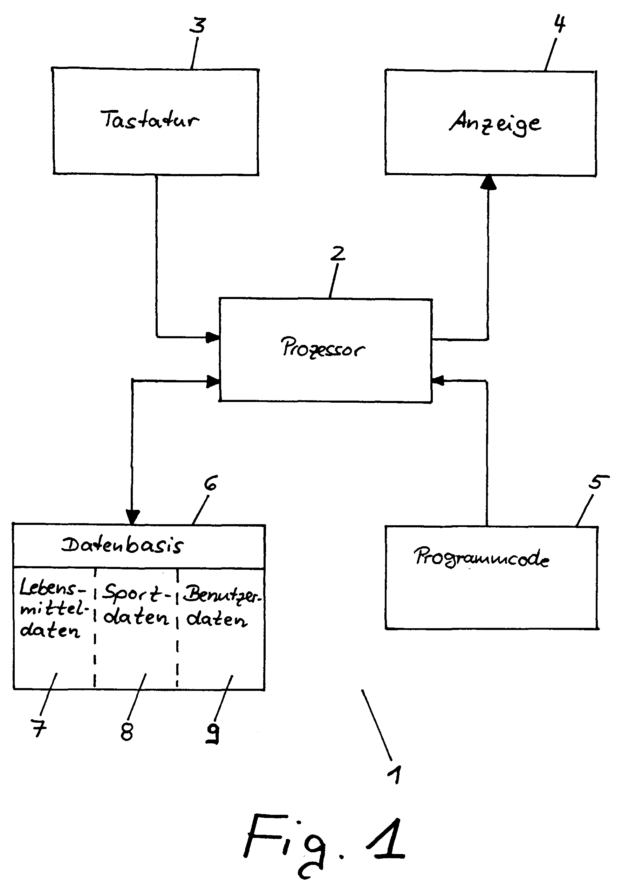

Das in Fig. 1 gezeigte Gerät umfaßt als zentralen Bestandteil einen Mikroprozessor 2, der über einen in einem entsprechenden Speicher 5 abgelegten Programmcode programmiert ist. Der als zentrale Steuereinheit dienende Prozessor 2 ist mit einem weiteren Speicher 6 verbunden, in dem die nachfolgend noch näher erläuterten Lebensmitteldaten und Sportdaten in Form entsprechender Datenbasen 7 bzw. 8 abgelegt sind. Des weiteren umfaßt der Speicher 6 eine Datenbasis 9 für persönliche Benutzerdaten, die von einem Benutzer über eine Tastatur 3 eingegeben bzw. von der Steuereinheit 2 berechnet worden sind. Die Steuereinheit 2 ist des weiteren mit einer Anzeigeneinheit 4 verbunden, über die Bedienhinweisen, Auswahlmöglichkeiten oder Warnmeldungen usw. ausgegeben werden können. Neben der visuellen Ausgabe einer Warnmeldung ist auch die akustische Ausgabe eines Warntons möglich.

In der Lebensmittel-Datenbasis 7 ist für eine Vielzahl unterschiedlicher Lebensmittel eine Kalorien- und Fettangabe je Lebensmitteleinheit gespeichert. In der Sportdatenbasis 8 ist für bestimmte sportliche Aktivitäten der dazugehörige Kalorienverbrauch abgelegt. Die in den Datenbasen 7 und 8 gespeicherten Einträge können von dem Benutzer über die Tastatur 3 beliebig ergänzt werden.

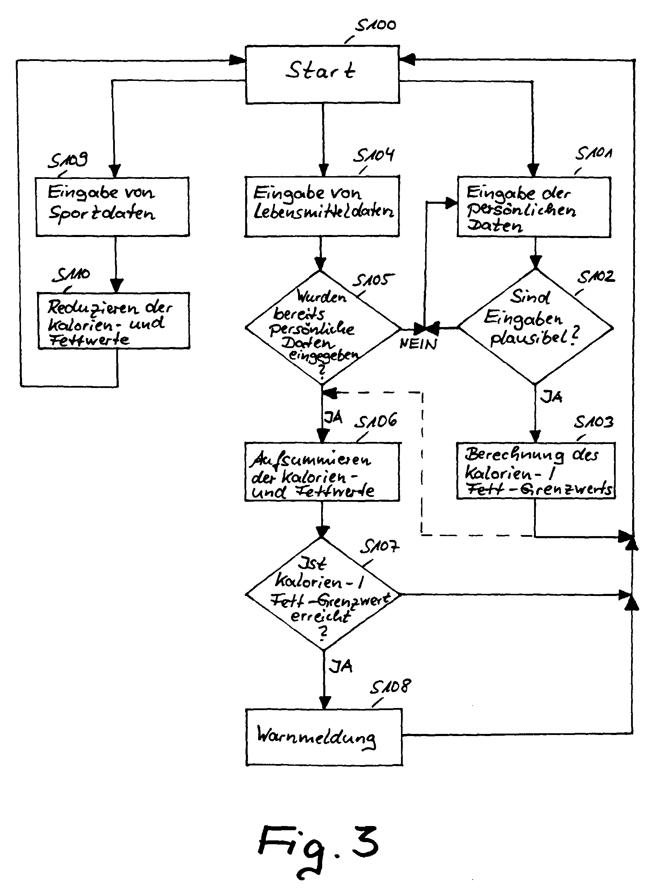

Nachfolgend soll die Betriebsweise des in Fig. 1 gezeigten Geräts anhand Fig. 3 erläutert werden.

Nach dem Einschalten des Gerätes (S100) wird dem Benutzer über die Anzeige 4 ein Menü mit den am häufigsten benutzten Informationen angeboten. Die weniger häufig genutzten Informationen sind in einem oder mehreren Untermenüs angesiedelt. Des weiteren ist es möglich, über bestimmte Funktionstasten der Tastatur 3 entsprechend zugeordnete Menüpunkte aufzurufen.

Zunächst wird davon ausgegangen, daß der Benutzer bestimmte persönliche Daten eingibt (S101). Diese persönlichen Daten werden vorzugsweise benutzerfreundlich von dem erfindungsgemäßen Gerät in Form entsprechender Eingabemasken abgefragt und können beispielsweise das Körpergewicht, daß Alter, das Geschlecht und den Lebensstil oder sonstige Lebenssituationsangaben umfassen. So kann beispielsweise der Benutzer auch Angaben über die von ihm hauptsächlich ausgeübte Tätigkeit (z.B. mäßig anstrengende Arbeit, leichte körperliche Arbeit oder starke körperliche Arbeit) oder über eine mögliche Schwangerschaft oder dergleichen eingeben. Die von dem Benutzer eingegebenen persönlichen Daten können nachträglich zu Korrekturzwecken geändert werden.

Während des Schritts S101 kann der Benutzer auch ein von ihm in einem angebbaren Zeitraum zu erreichendes Gewicht angeben. Vorzugsweise kann als Gewichtsziel das Idealgewicht, das Normalgewicht oder ein selbst definiertes Wunschgewicht ausgewählt werden. Anschließend wird eine Plausibilitätsüberprüfung durchgeführt (S102), wobei insbesondere überprüft wird, ob das von dem Benutzer eingegebene angestrebte Gewicht und der ebenfalls hierzu angegebene Zeitraum realistisch sind. Ist dies nicht der Fall, wird erneut die Eingabemaske für die Eingabe des Sollgewichts aufgerufen.

Wurden auf diese Weise die persönlichen Daten des Benutzers erfaßt, berechnet das Gerät bzw. die Steuereinheit 2 anhand der eingegebenen persönlichen Daten die zur Erzielung des angestrebten Sollgewichts maximal zulässige tägliche Kalorienzahl/Fettmenge (S103). Dieser somit berechnete Grenzwert wird über die Anzeige 4 ausgegeben.

Über die Tastatur 3 können jederzeit die die Kalorienzahl und/oder Fettmenge eines verzehrten Lebensmittels betreffenden Angaben eingegeben werden (S104). Dies ist insbesondere deshalb möglich, da es sich bei dem vorliegenden Gerät um ein tragbares Gerät in Handtaschenformat handelt, wodurch eine größtmögliche Mobilität gewährleistet ist. Die Eingabe der Lebensmitteldaten (S104) erfolgt in der Regel anhand der in der Lebensmitteldatenbasis 7 gespeicherten Vorschlagsliste, in der für verschiedene Lebensmittel Kalorien- und Fettangaben je Lebensmitteleinheit gespeichert sind. Der Benutzer kann ein bestimmtes gespeichertes Lebensmittel auswählen und durch eine entsprechende Mengenangabe die in der Lebensmitteldatenbasis 7 gespeicherten Kalorien- und Fettangaben entsprechend seinem tatsächlichen Verzehr anpassen. Anstelle der Auswahl aus der aus der Lebensmitteldatenbasis 7 gespeicherten Vorschlagsliste kann der Benutzer auch lediglich eine verzehrte Kalorienzahl/Fettmenge ohne Angabe des entsprechenden Lebensmittels eingeben. Des weiteren ist, wie bereits erwähnt worden ist, auch möglich, die in der Lebensmitteldatenbasis gespeicherte Vorschlagsliste benutzerspezifisch zu ergänzen bzw. zu erweitern, um zusätzliche, bisher nicht gespeicherte Lebensmittel in die Lebensmitteldatenbasis aufzunehmen. Die in der Lebensmitteldatenbasis 7 gespeicherten Daten können von dem Benutzer jederzeit zu Informationszwecken abgerufen werden.

Bei der Eingabe der verzehrten Lebensmittel bzw. der entsprechenden Kalorienzahl und der entsprechenden Fettmenge wird zusätzlich überprüft, ob bereits die zur Berechnung des täglichen Kalorien-/Fettgrenzwerts erforderlichen persönlichen Daten eingegeben worden sind (S105). Ist dies nicht der Fall, wird automatisch in das entsprechende Menü verzweigt und nach Eingabe der persönlichen Daten sowie der Berechnung des Kalorien-/Fettgrenzwerts (S103) wieder zu dem ursprünglichen Menüpunkt zurückgesprungen.

Anschließend wird die dem verzehrten Lebensmittel entsprechende Kalorienzahl/Fettmenge über den Tag aufsummiert (S106), um somit die bisher über den Tag aufgelaufene Gesamtmenge an Kalorien und Fett zu ermitteln. Diese aufsummierte Gesamtmenge wird dann mit dem im Schritt S103 berechneten Grenzwert verglichen, um feststellen zu können, ob sich der Benutzer das verzehrte Lebensmittel hinsichtlich seines angestrebten Sollgewichts noch 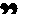 leisten" kann oder nicht (S107). Wurde der berechnete Kalorien-/Fettgrenzwert erreicht, wird eine Warnmeldung ausgegeben (S108), wobei dies optisch (über die Anzeige 4) oder akustisch (durch Ausgabe eines Warntons über einen Lautsprecher) erfolgen kann.

Die von dem Benutzer eingegebenen Lebensmitteldaten bzw. die entsprechenden Kalorien- und Fettinformationen werden zusammen mit den zuvor eingegebenen persönlichen Daten in der in Fig. 1 gezeigten Benutzerdatenbasis 9 gespeichert und sind jederzeit abrufbar, so daß der Benutzer auch nachträglich noch die täglich verzehrten Kalorien über einen bestimmten Zeitraum (z.B. über eine Woche) betrachten kann.

Neben der Eingabe der persönlichen Daten (S101) und der Lebensmitteldaten (S104) können von dem Benutzer auch Sportdaten eingegeben werden (S109), die die von dem Benutzer durchgeführten sportlichen Aktivitäten beschreiben. Die Eingabe der Sportdaten erfolgt ähnlich zu der Eingabe der Lebensmitteldaten, wobei auf die in der Sportdatenbasis 8 gespeicherte Vorschlagsliste zugegriffen wird, so daß der Benutzer eine als Eintrag in diese Vorschlagsliste gespeicherte sportliche Aktivität auswählen und ggf. die vorgegebene Zeitdauer dieser sportlichen Betätigung anpassen kann. Vorzugsweise wird analog zu dem Schritt S105 auch nach der Eingabe der Sportdaten überprüft, ob bereits die persönlichen Daten eingegeben worden sind, um dann ggf. in das Eingabemenü für die persönlichen Daten zu verzeigen.Nach Auswahl einer entsprechenden sportlichen Betätigung wird die im Schritt S106 berechnete Tagessumme der Kalorien- und Fettwerte entsprechend dem Kalorienverbrauch der sportlichen Betätigung reduziert (S110). Die Sportdatenbasis 8 kann ähnlich zu der Lebensmitteldatenbasis 7 durch den Benutzer ergänzt werden, um weitere Einträge in die Sportdatenbasis 8 aufzunehmen. Anstelle der Auswahl der vorgegebenen sportlichen Betätigung ist es auch möglich, direkt einen bestimmten Kalorienverbrauch einzugeben.

Da die von dem Benutzer eingegebenen Lebensmitteldaten sowie die während des Betriebs des Gerätes berechneten Kalorien- und Fettsummenwerte über einen längeren Beobachtungszeitraum in der in Fig. 1 gezeigten Benutzerdatenbasis 9 gespeichert werden, kann der Benutzer die darin gespeicherten Informationen jederzeit abrufen, wobei Tages-, Wochen- oder Jahresbilanzen erstellt werden können. So kann beispielsweise nachträglich überprüft werden, wie viele Kalorien bzw. wieviel Fett und welche Lebensmittel in den letzten Tagen verzehrt oder welche sportliche Aktivitäten durchführt worden sind. Darüber hinaus kann tageweise aufgeführt werden, wie viele Kalorien oder wieviel Fett abzüglich der sportlichen Aktivitäten in der aktuellen Woche verzehrt worden sind oder wie die Gewichtsentwicklungin der aktuellen Woche oder in den vorhergehenden Wochen war, um die Gewichtsabnahme überprüfen zu können. Die von dem Benutzer angegebenen Daten können jederzeit vollständig oder teilweise gelöscht werden, um einen Neuanfang zu starten. Insbesondere ist es möglich, lediglich die Daten des aktuellen Tages zu löschen, um Fehleingaben korrieren zu können. Umständlich zu bedienende Änderungs- oder Korrekturfunktionen können auf diese Weise vermieden werden.

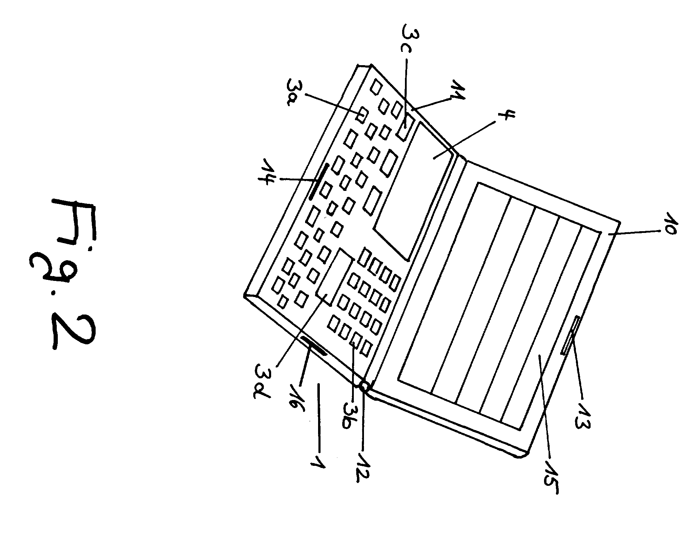

In Fig. 2 ist eine perspektivische Außenansicht eines bevorzugten Ausführungsbeispiels der vorliegenden Erfindung dargestellt, welches dem anhand Fig. 1 oder Fig. 3 erläuterten Ausführungsbeispiels entspricht.

Das in Fig. 2 dargestellte Gerät 1 umfaßt ein aufklappbares Gehäuse, dessen beide Gehäuseteile 10,11 beispielsweise über ein Gelenk 12 miteinander verbunden sind. Im unteren Gehäuseteil 11 ist der eigentliche Rechner mit der in Fig. 1 dargestellten Steuereinheit 2, der Tastatur 3, der Anzeige 4 sowie den einzelnen in Fig. 1 dargestellten Speichern 5,6 untergebracht. Die Speicher können derart dimensioniert sein, daß einige MB-Daten und einige KB-Programmcode gespeichert werden können. Die Tastatur umfaßt eile alphanumerische Tastatur 3a mit einem Zifferntastenblock 3b.Darüber hinaus umfaßt die Tastatur Funktionstasten 3c, die vorzugsweise von dem Benutzer beliebig programmierbar sind und zum Aufrufen besonders häufig benutzter Menüpunkte vorgesehen sind. Des weiteren ist eine drucksensitive Eingabefläche 3d vorgesehen, über die ergänzend zur eigentlichen Tastatur mit den Tasten 3a, 3b, 3c mit Hilfe eines Eingabestifts Eingaben getätigt werden können. Zudem können Curser-Steuerungstasten vorgesehen sein, um beispielsweise zur Auswahl eines auf der Anzeigeneinheit 4 dargestellten Listenelements einen Curser an eine gewünschte Stelle bewegen zu können, wobei nach Auswahl des Listenelements der ausgewählte Eintrag vorzugsweise graphisch hervorgehoben dargestellt wird. Die Anzeigeneinheit 4 kann durch ein mehrzeiliges monochromes Display (z.B. mit sieben Zeilen à 35-40 Zeichen) gebildet sein, welches ggf. mit einer Hintergrundbeleuchtung versehen ist.

Das Gerät wird als ein lediglich auf die Ernährungskontrolle spezialisiertes Gerät angeboten und entspricht somit keinem Universalcomputer. Durch die Spezialisierung der Ernährungskontrolle können für den Benutzer verwirrende und unnötige funktionelle Tätigkeiten vermieden werden, und das Gerät wird billiger und leichter bedienbar. Die Gehäuseabmessungen des Geräts können beispielsweise 8 x 15cm betragen. Die Abmessungen sind auf jeden Fall derart zu wählen, daß das Gerät Taschenformat aufweist und ähnlich zu einem Taschenorganizer in jede Handtasche paßt. Die Stromversorgung des Geräts 1 erfolgt vorzugsweise über Batterie/Akku, wobei für den Fall eines Batteriewechsels zur Vermeidung von Datenverlusten eine/ein Pufferbatterie/Pufferakku vorgesehen sein kann. Des weiteren weist das Gerät 1 eine externe Schnittstelle 16 auf, über die das Gerät 1 an einen Rechner zur Datensynchronisation bzw. Datenaustausch angeschlossen werden kann. Auf diese Weise können z.B. die in Fig. 1 gezeigten Lebensmittel- und Sportdatenbasen 7,8 durch externe Dateneinspeisung aktualisiert werden. Ebenso ist möglich, über die Schnittstelle 16 die in der Datenbasis 9 gespeicherten Benutzerdaten an einen weiteren Rechner zu übertragen, wo sie ausgewertet und betrachtet werden können.

Die beiden Gehäusehälften 10,11 des in Fig. 2 gezeigten Geräts (1) können über einen Schnappmechanismus im zusammengeklappten Zustand miteinander verriegelt werden, wobei zu diesem Zweck die obere Gehäusehälfte 10 einen Vorsprung 13 aufweist, der in eine entsprechend ausgebildete Ausnehmung 14 der unteren Gehäusehälfte 11 eingreift. In der oberen Gehäusehälfte 10 ist ein Handbuch 15 bzw. ein entsprechendes Blatt mit Bedieninformationen integriert, so daß nach Aufklappen der oberen Gehäusehälfte 10 der Benutzer jederzeit der oberen Gehäusehälfte bzw. dem Deckel 10 die Bedieninformationen entnehmen kann. Diese Möglichkeit ist insbesondere gegenüber einer aufwendigen softwaremäßigen Hilfefunktion vorteilhaft, da beispielsweise auch ein EDV-Laie leicht auf die Bedieninformationen zugreifen kann.

Ungeachtet dessen kann jedoch das Gerät auch zusätzlich mit einer derartigen Software-Hilfefunktion ausgerüstet sein. Das in der oberen Gehäusehälfte 10 angebrachte Handbuch 15 kann neben Bedienhinweisen auch Erläuterungen zur Diätproblematik oder dgl. enthalten. An der Geräteaußenseite ist vorzugsweise kein Schriftzug angebracht, der darauf schließen lassen könnte, daß das Gerät 1 zur Ernährungskontrolle oder Diätkontrolle eingesetzt wird.

Die in Fig. 1 gezeigte Steuereinheit 2 führt die einzelnen zuvor beschriebenen Berechnungen anhand allgemein bekannter Algorithmen, wie z.B. dem BMI-Algorithmus (Body Mass Index) oder dem Broca-Algorithmus, durch, die insbesondere durch den in dem Speicher 5 abgelegten Programmcode definiert sind. Das Gerät ist durch Anpassung/Austausch der Datenbasen 7-9 sowie der durch den Programmcode im Speicher 5 festgelegten Ausgabetexte an länderspezifische Besonderheiten leicht anpaßbar. Demzufolge sollte es sich bei den Speichern 5 und 6 um austauschbare oder leicht wiederbeschreibbare Speicher handeln. Darüber hinaus ist auch denkbar, für unterschiedliche Betriebssprachen unterschiedliche Datenbasen vorzusehen, so daß die Steuereinheit 2 nach einer beispielsweise menügesteuerten Auswahl der Betriebssprache auf die der ausgewählten Betriebssprache entsprechende Datenbasen zugreifen kann.

## Patentansprüche

1. Vorrichtung zur Ernährungskontrolle,
mit Eingabemitteln (3) zum Eingeben von persönlichen Daten eines Benutzers sowie Lebensmitteldaten, welche von dem Benutzer verzehrte Lebensmittel beschreiben,
mit Steuermitteln (2) zum Erfassen der eingegebenen persönlichen Daten und der Lebensmitteldaten, zum Aufsummieren von mindestens einem den eingegebenen Lebensmitteldaten entsprechenden ernährungsspezifischen Kennwert der verzehrten Lebensmittel über ein bestimmtes Zeitintervall zur Bestimmung einer Kennwertsumme und zum Feststellen, ob die über das Zeitintervall bestimmte Kennwertsumme eine einen den persönlichen Daten entsprechenden Grenzwert erreicht, und
mit Ausgabemitteln (4) zum Ausgeben einer Warnung an den Benutzer, falls die Steuermittel (2) ein Erreichen des Grenzwerts durch die Kennwertsumme festgestellt haben.

2. Vorrichtung nach Anspruch 1,
**dadurch gekennzeichnet,**
daß der mindestens eine ernährungsspezifische Kennwert die Kalorienzahl und/oder die Fettmenge der von dem Benutzer verzehrten Lebensmittel beschreibt.

3. Vorrichtung nach Anspruch 1 oder 2,
**dadurch gekennzeichnet,**
daß die Ausgabemittel eine Anzeigeneinheit (4) umfassen, und
daß die Eingabemittel (3) derart ausgestaltet sind, daß von dem Benutzereingegebene Daten im Sinne einer hierarchischen Menüsteuerung abfragbar sind.

4. Vorrichtung nach Anspruch 3,
**dadurch gekennzeichnet,**
daß die Eingabemittel (3) und die Anzeigeneinheit (4) derart zusammenwirken,
daß die persönlichen Daten und die Lebensmitteldaten über eine graphische Benutzerführung eingebbar sind, wobei mit Hilfe der Eingabemittel (3) zwischen verschiedenen auf der Anzeigeneinheit (4) dargestellten Wahloptionen ausgewählt werden kann.

5. Vorrichtung nach Anspruch 3 oder 4,
**dadurch gekennzeichnet,**
daß die Eingabemittel (3) eine Tastatur umfassen, und
daß die Tastatur (3) Funktionstasten (3c) zum Aufrufen häufig verwendeter Menüpunkte aufweist.

6. Vorrichtung nach einem der vorhergehenden Ansprüche,
**dadurch gekennzeichnet,**
daß die Eingabemittel (3) und die Steuermittel (2) derart ausgestaltet sind, daß als persönliche Daten des Benutzers das Alter, das Geschlecht, das augenblickliche Körpergewicht und/oder die Lebenssituation des Benutzers beschreibende Angaben eingebbar sind.

7. Vorrichtung nach einem der vorhergehenden Ansprüche,
**gekennzeichnet durch**
erste Speichermittel (7) zum Speichern einer Lebensmitteldatenbasis, in der zu bestimmten Lebensmitteln die entsprechenden ernährungsspezifischen Kennwerte abgelegt sind.

8. Vorrichtung nach Anspruch 7,
**dadurch gekennzeichnet,**
daß in den ersten Speichermitteln (8) die ernährungsspezifischen Kennwerte pro einer Lebensmitteleinheit für verschiedene Lebensmittel gespeichert sind und
daß die Eingabemittel (3) und die Steuermittel (2) derart ausgestaltet sind, daß aus der in den ersten Speichermitteln (7) gespeicherten Lebensmitteldatenbasis ein bestimmtes von dem Benutzer verzehrtes Lebensmittel auswählbar und die tatsächlich von dem Benutzer verzehrten Lebensmitteleinheiten eingebbar sind.

9. Vorrichtung nach einem der vorhergehenden Ansprüche,
**gekennzeichnet durch**
zweite Speichermittel (8) zum Speichern einer Sportdatenbasis, in der für bestimmte sportliche Betätigungen die dabei verbrauchte Kalorien- und/oder Fettmenge abgelegt ist.

10. Vorrichtung nach Anspruch 2 und einem der vorhergehenden Ansprüche,
**dadurch gekennzeichnet,**
daß die Steuermittel (2) derart ausgestaltet sind, daß sie bei der Berechnung einer Gesamtkalorien- und/oder Gesamtfettmenge als Kennwertsumme eine sportliche Betätigung des Benutzers, welche in Form von Sportdaten über die Eingabemittel (3) eingegeben worden ist, berücksichtigen.

11. Vorrichtung nach Anspruch 9 und Anspruch 10,
**dadurch gekennzeichnet,**
daß die sportliche Betätigung des Benutzers durch Auswahl eines Eintrags der in den zweiten Speichermitteln (8) gespeicherten Sportdatenbasis eingebbar ist.

12. Vorrichtung nach Anspruch 7 oder 8 oder Anspruch 9 oder 11,
**dadurch gekennzeichnet,**
daß die in den ersten Speichermitteln (7) und/oder zweiten Speichermitteln (8) gespeicherten Einträge der Lebensmitteldatenbasis bzs. Sportdatenbasis über die Eingabemittel (3) abfragbar und über die Ausgabemittel (4) ausgebbar sind.

13. Vorrichtung nach einem der vorhergehenden Ansprüche,
**dadurch gekennzeichnet,**
daß die Steuermittel (2) derart ausgestaltet sind, daß sie den mindestens einen ernäbrungsspezifischen Kennwert der von dem Benutzer verzehrten Lebensmittel über einen ganzen Tag aufsummieren.

14. Vorrichtung nach Anspruch 2 und einem der vorhergehenden Ansprüche,
**dadurch gekennzeichnet,**
daß die Steuermittel (2) und die Eingabemittel (3) derart ausgestaltet sind, daß als persönliche Daten ein von dem Benutzer angestrebtes Körpergewicht und ein Zeitraum, über den der Benutzer das angestrebte Körpergewicht ausgehend von seinem augenblicklichen Körpergewicht erreichen will, eingebbar sind, wobei die Steuermittel (2) abhängig von dem angestrebten Körpergewicht und dem entsprechend eingegebenen Zeitraum sowie weiteren eingegebenen persönlichen Daten des Benutzers den Grenzwert für eine der Kennwertsumme entsprechende Gesamtkalorien- und/oder Gesamtfettmenge ermitteln.

15. Vorrichtung nach Anspruch 14,
**dadurch gekennzeichnet,**
daß die Steuermittel (2) derart ausgestaltet sind, daß sie bei der Eingabe des angestrebten Körpergewichts und des dafür vorgesehenen Zeitraums eine Plausiblitätsprüfung durchführen und unrealistische Eingaben nicht akzeptieren.

16. Vorrichtung nach einem der vorhergehenden Ansprüche,
**gekennzeichnet durch**
dritte Speichermittel (9) zum Speichern der von dem Benutzer eingegebenen Lebensmitteldaten, der von dem Benutzer eingegebenen persönlichen Daten und/oder der von den Steuermitteln (2) bestimmten Kennwertsumme über die ernährungsspezifische Kennwerte der von dem Benutzer verzehrten Lebensmittel, wobei die in den dritten Speichermitteln (9) gespeicherten Daten zusammengefaßt zu bestimmten Beobachtungszeiträumen abfragbar und über die Ausgabemittel (4) ausgebbar sind.

17. Vorrichtung nach einem der vorhergehenden Ansprüche,
**dadurch gekennzeichnet,**
daß die Vorrichtung in Form eines tragbaren Geräts (1) in Taschenformat ausgestaltet ist.

18. Vorrichtung nach Anspruch 17,
**dadurch gekennzeichnet,**
daß das Gerät (1) in einem in zwei Hälften (10,11) aufklappbaren Gehäuse untergebracht ist, wobei das Gerät (1) im aufgeklappten Zustand des Gehäuses auf der einen Gehäusehälfte (11) bedient werden kann, während die andere Gehäusehälfte (10) einen Informationsträger (15) mit Bedieninformationen zur Bedienung des Gerätes (1) aufweist, wobei die Bedieninformationen im aufgeklappten Zustand für den Benutzer sichtbar sind.

19. Vorrichtung nach einem der vorhergehenden Ansprüche,
**dadurch gekennzeichnet,**
daß die Vorrichtung eine Schnittstelle (16) zum Empfang von extern zugeführten Daten bzw. zum Übertragen von gespeicherten Daten an einen daran anschließbaren Rechner aufweist.

20. Vorrichtung nach einem der Ansprüche 7-9, 11 oder 12,
**dadurch gekennzeichnet,**
daß die ersten bzw. zweiten Speichermittel (7,8) austauschbar sind.
